(19) Europäisches Patentamt
European Patent Office
Office européen des brevets

(11) **EP 2 767 224 B1**

(12) **EUROPEAN PATENT SPECIFICATION**

(45) Date of publication and mention
of the grant of the patent:
**01.03.2017   Bulletin 2017/09**

(51) Int Cl.:
***A61B 5/00*** *(2006.01)*      ***A61B 5/0472*** *(2006.01)*
***A61N 1/36*** *(2006.01)*

(21) Application number: **14153174.9**

(22) Date of filing: **30.01.2014**

(54) **A method for the detection of subcutaneous cardiac signals and a cardiac device for use in detecting subcutaneous cardiac signals**

Verfahren für den Nachweis von subkutanen Herzsignalen und Herzvorrichtung zum Nachweis von subkutanen Herzsignalen

Procédé pour la détection de signaux cardiaques sous-cutanés et dispositif cardiaque sous-cutané destiné à être utilisé dans la détection de signaux cardiaques

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **19.02.2013   US 201361766137 P**

(43) Date of publication of application:
**20.08.2014   Bulletin 2014/34**

(73) Proprietor: **BIOTRONIK SE & Co. KG**
**12359 Berlin (DE)**

(72) Inventors:
• **Moulder, J. Christopher**
**Portland, OR Oregon 97202 (US)**

• **de Voir, Christopher S.**
**Tigard, OR Oregon 97223 (US)**
• **Whittington, R. Hollis**
**Portland, OR Oregon 97202 (US)**
• **Garner, Garth**
**Tigard, OR Oregon 97224 (US)**

(74) Representative: **Galander, Marcus et al**
**Biotronik SE & Co. KG**
**Corporate Intellectual Properties**
**Woermannkehre 1**
**12359 Berlin (DE)**

(56) References cited:
**US-A- 4 458 692     US-A- 5 967 994**

EP 2 767 224 B1

**Description**

The invention relates to the sensing of electrocardiogram (ECG) signals, in particular to the sensing of subcutaneous ECG signals.

The QRS complex is a specific sequence of deflections that can be detected within an electrocardiogram (ECG) signal, measured from the body of a living being. It represents the depolarization of the right and left ventricles of the heart. An ECG signal shows five deflections, or waves, that are arbitrarily named P, Q, R, S and T. In an ECG signal, Q waves are represented as small downward deflections following the P wave, and the R wave is represented as a large upward deflection, or as a spike. The S wave represents a small downward deflection following the R wave. The time intervals between QRS complexes indicate the heart rate of the heart of a living being. The QRS complex of a healthy human being lasts for a time interval between 60 to 120 milliseconds. Wide QRS complexes, or those lasting longer than 120 milliseconds, indicate ventricle impairment such as bundle branch blocks. Furthermore often with bundle branch blocks, a second upward deflection occurs within the QRS complex. Other causes for a wide QRS complex include ectopic foci or abnormal pacemaker sites that slow conduction within the heart and increase the depolarization time of the heart muscle. Moreover, other deviations of a patients QRS complex from a normal one can indicate various symptoms for diseases or malfunctions of different parts or areas of, for example, a human heart.

US 4458692 A teaches a system for predicting ventricular tachycardia from electrocardiograph input waveforms, wherein a first means generates a signal representative of the input waveforms exceeding a preselected level and a second filter means has its gain controlled responsive to the first means output signal, and provides an output responsive to said input waveforms.

US 5967994 A teaches a method and system for characterizing the quality of signals indicative of heart function, where such signals indicative of heart function are derived from electrocardiographic measurements.

It has always been a crucial issue of cardiac research to enable precise detection, analysis, and evaluation of a patient's QRS complex. Therefore, a method is provided which enables a highly accurate detection of a QRS complex with an increased signal-to-noise (SNR) ratio, compared to methods disclosed within the state of the art.

According to at least one embodiment, a method for the detection of QRS signals sensed by pairs of electrodes is provided. The method comprises the steps of: providing at least two input heart signals via at least two separate input channels, each channel connected to external sensing electrodes, wherein each of the at least two input heart signals is recorded by pairs of sensing electrodes that have one electrode in common and provided coincidental in time; generating estimated signals from the input heart signals; combining the estimated signals to a combined input stream; detecting the QRS signal by comparing the combined input stream to an adaptive detection threshold which adapts throughout time. The estimated signals comprise spatially derived estimates and/or temporally derived estimates.

The advantage of the method is that by the use of spatial and temporal estimates for the combination of input heart signals provided by different input channels, the signal to noise ratio is increased by smoothing some of the random noise associated with each input channel, while emphasizing the QRS complexes measured from the combined input stream. This method is particularly advantageous when of the pairs of sensing electrodes one electrode is shared by two of the input channels. Moreover, via the adaptation of the adaptive detection threshold, the detection criteria for the detection of a QRS complex is adjusted to the behavioral characteristics of the heart of a living being.

In a preferred embodiment, the temporally derived estimate for an input channel is calculated as a smoothed value calculated from samples of the input heart signal of an input channel. In one embodiment, an average value is calculated using samples of the input heart signal of an input channel that are taken before and after a current sample of the input heart signal of the respective input channel. In such an embodiment, random noise is smoothened in each of the input channels.

In a preferred embodiment, the spatially derived estimate of an input channel is calculated as combination of the input heart signals provided by the other input channel(s).

The calculation of the spatially derived estimates is possible since for these at least two input channels the heart signals are gathered by pairs of sensing electrodes for each channel, where any two channels share one electrode. A spatially derived estimate of any input channel can be made from the at least two input channels, thereby reducing the influence of local electrode noise.

Preferably the step of combining the estimated signals to a combined input stream further comprises the step of combining the spatially derived estimate, the temporally derived estimate and optionally the input heart signal of an input channel for each input channel respectively to provide a composite estimate signal for each input channel respectively.

To reduce the probability that the combining will produce a null result, one composite channel may be augmented before the final combination. In a further embodiment, a prospect is calculated for each of the input channels respectively, using the composite estimate signals, wherein within the calculation of a certain prospect for a channel, the respective composite estimate signal calculated for the respective channel is augmented.

The concept of prospect allows for the selection of at least one prospect from the combined input streams according to selectable criteria such as peak or maximum signal amplitude or signal-to-noise ratio.

In a preferred embodiment, the maximum signal amplitude of the prospect is the selection criteria.

In one preferred embodiment, the at least two input heart signals or the combined input stream, or both, are filtered with a high-pass, low-pass, or a pass-through filter, or any combination thereof Preferably, at least two filters are arranged in series or at least two filters form a filter loop structure. Preferably the net pass band output by the filters arranged in series or by at least two filters that form a filter loop structure is between 1 and 50 Hz.

Preferably the adaptive detection threshold is set after each detection of a QRS signal, wherein the value of the adaptive detection threshold is initially set as a percentage of a QRS-peak amplitude of the combined input stream measured during a sensing cycle and wherein the value of the adaptive detection threshold is initially set as a different percentage of a QRS-peak amplitude of the combined input stream measured during a detection of a QRS signal and wherein the adaptive detection threshold decreases throughout time.

Preferably, the adaptive detection threshold decreases in steps, for example by an absolute, relative, or adaptive value until it reaches a target threshold, wherein the value of the target threshold is initially set as a percentage of a QRS-peak amplitude of the combined input stream measured during a detection of a QRS signal and wherein the adaptive detection threshold and the target threshold are always greater than or equal to a minimum threshold. Within such an embodiment, the adaptive detection threshold and the target threshold are adjusted for every QRS-peak detected, which significantly increases detection accuracy.

Preferably the duration of a step is adjusted based on a moving average of the ventricular cycle length measured from the composed heart signal of the body from which the combined input stream is measured. In such an embodiment it is possible to control the adaptive detection threshold or the target threshold so that it remains above the minimum threshold, since the steps are adjusted such that the target threshold is above or equal to the minimum threshold. Adjusting the threshold decay also prevents noise detection by maintaining the detection threshold as high as possible. The detection threshold is adjusted to detect the next expected QRS complex while avoiding noise between the complexes, such as the T-wave.

In a preferred embodiment, the adaptive detection threshold is set to the minimum threshold based on the last detected QRS complex after a predetermined time. In such an embodiment, QRS-peaks with a low amplitude can be detected which allows a precise analyses of the heartbeat characteristics of a living being.

Preferably the minimum threshold is determined by a vector residual of the composite estimate signals. In one embodiment, an error $\varepsilon$ is calculated, filtered and used for the determination of the minimum threshold. The calculation of error $\varepsilon$ depends on the arrangement of the electrodes and which electrode is the reference for each channel. Error $\varepsilon$ is calculated that it is zero if no noise is present and a value greater than zero represents the noise floor. In one embodiment, $\varepsilon$ is equal to $\varepsilon = A_{est} - B_{est} - C_{est}$. In such an embodiment, also the minimum threshold dynamically depends on the composite estimate signals. By setting the minimum threshold to a vector residual equal to a noise error, it can be assured that only QRS-peaks with amplitudes that are greater than the noise error are detected.

Preferably a low signal flag (LSF) is set if a QRS-peak amplitude of the combined input stream, which is measured during a detection of a QRS signal, is lower than a scaled value of the minimum threshold. In such an embodiment, a low signal flag is set or declared when the peak of a detected QRS complex is too low to be considered reliable, which could happen if the QRS complex has low amplitude, noise has a large amplitude, or if noise caused the detection. In such an embodiment, the reliability of the method is increased.

Furthermore, a cardiac device for use in detecting QRS signals within a composite heart signal of a body is provided, comprising an input unit to which at least three external sensing electrodes are attachable to provide at least two input heart signals recorded by pairs of sensing electrodes via at least two separate input channels provided coincidental in time. The cardiac device comprises a estimation unit, configured to generate estimated signals from the input heart signals; a combination unit, configured to combine the three input heart signals and the estimated signals to a combined input stream; an automatic sense control unit, configured to compare the combined input stream to an adaptive detection threshold which adapts throughout time and a system adjustment unit, configured to adjust at least one parameter of the automatic sense control unit. The cardiac device is configured to execute the method according to the present invention. With such a cardiac device, the method is usable within a cardiac device, so that the advantages mentioned for the method also apply to the cardiac device. Preferably, the system adjustment unit is configured to adjust parameters related to sensing, noise and timing of the automatic sense control unit.

Preferably the estimation unit, the combination unit, the automatic sense control unit, and the system adjustment unit are realized within a digital signal processor. It is possible to implement a large variety of applications within a compact digital signal processor or a digital signal processing unit.

Figure 1          shows a high level block diagram of an embodiment of a cardiac device and a corresponding method

Figures 2A and 2B   shows input channel orientations of embodiments of a cardiac device

| Figure 3 | shows a schematic illustration of the combination of three input heart signals to a combined input stream |
|---|---|
| Figure 4 | shows a schematic illustration of the general QRS-peak detection flow |
| Figure 5 | shows a schematic illustration of a threshold adjustment for large amplitude QRS-peaks according to one exemplary embodiment |
| Figure 6 | shows a timing diagram for sense and noise markers settable by the cardiac device according to one exemplary embodiment and an exemplary combined input stream showing a QRS complex without noise followed by a QRS complex with noise |
| Figure 7 | shows intervals used for average cycle length calculation according to one exemplary embodiment |
| Figure 8 | shows a schematic illustration of a threshold adjustment of the adaptive detection threshold for small amplitude QRS-peaks according to one exemplary embodiment |

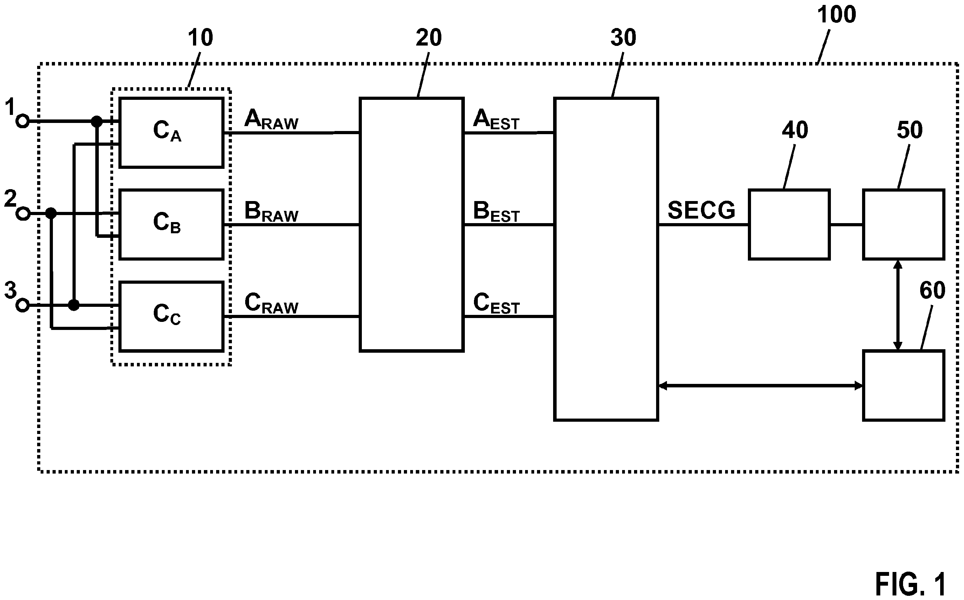

Figure 1 shows a high level block diagram of an embodiment of a cardiac device 100. A method is executable by the exemplary embodiment of the cardiac device 100. The exemplary device and associated method are designed to make use of at least two sensing vectors in order to increase noise immunity and signal detection accuracy.

The implantable medical device 100 records the electrical activity of the heart using at least three electrodes that may be placed on the housing of the device. The device is preferably implanted subcutaneously.

Referring to Figure 1, the implantable medical device 100 comprises an input unit 10 that is connected to the electrodes 1, 2 and 3. Pairs of the electrodes 1, 2 and 3 are connected to the input channels $C_A$, $C_B$ and $C_C$. Such input units are known in the art and may comprise amplifiers and analog-digital converters. At the output of the input unit 10 three input heart signals $A_{raw}$, $B_{raw}$ and $C_{raw}$ are provided coincidental in time.

In the input unit 10 the input channels $C_A$, $C_B$ and $C_C$ optionally comprise signal filtering in hardware or software. For example, the signals are filtered with a high-pass first order analog Butterworth filter that provides a corner frequency of 1 Hz. Subsequently, all input channels $C_A$, $C_B$ and $C_C$ are optionally filtered with a low-pass filter that provides a corner frequency at 50 Hz to remove any high frequency noise.

The input heart signals $A_{raw}$, $B_{raw}$ and $C_{raw}$ are input to an estimation unit 20 to generate estimated signals from the input heart signals $A_{raw}$, $B_{raw}$ and $C_{raw}$. The estimated signals comprise temporally derived estimates, spatially derived estimates or combinations thereof.

In one embodiment, the estimation unit 20 calculates temporally derived estimates $A_{temporal}$, $B_{temporal}$ and $C_{temporal}$ for each input channel $C_A$, $C_B$ and $C_C$. The temporally derived estimates $A_{temporal}$, $B_{temporal}$ and $C_{temporal}$ are each equal to an average value calculated from samples of the respective input heart signal $A_{raw}$, $B_{raw}$ and $C_{raw}$ of an input channel $C_A$, $C_B$ and $C_C$, taken before and after a current sample of the input heart signal $A_{raw}$, $B_{raw}$ and $C_{raw}$ of the respective channel $C_A$, $C_B$ and $C_C$ was taken. Expressed in other words, the calculation of a temporally derived estimate $A_{temporal}$, $B_{temporal}$ and $C_{temporal}$ is performed by an averaging filter with no memory that removes some high frequency noise. The temporally derived estimate $A_{temporal}$, $B_{temporal}$ and $C_{temporal}$ uses samples before and after the current sample to produce an average value. This average value is the predicted value of the sample in time, which can be calculated for example by the following equation:

$$y[n-1] = \frac{x[n] + x[n-2]}{2},$$

wherein y[n-1] is the temporal estimate of a single input channel delayed by one sample, x[n] is the current sample and x[n-2] is a previous sample delayed by two while n is the relative sample number. The effect of this operation delays the output by 1 sample. Alternatively the average value can be calculated using more samples, a moving average filter, or by other functions such as the CDF(2,2) wavelet smoothing function for example.

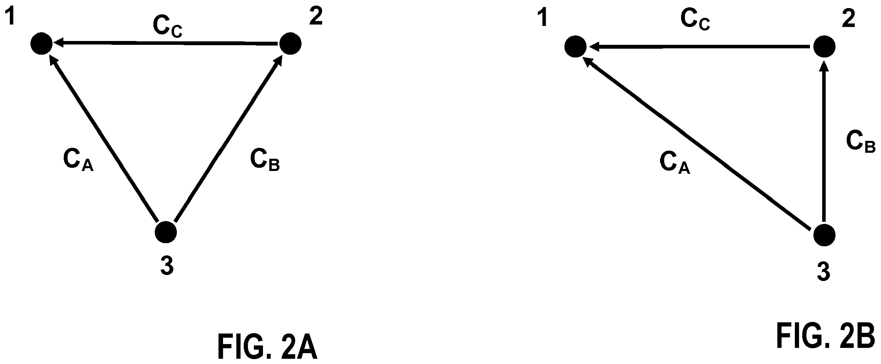

The estimation unit 20 also calculates the spatially derived estimate of an input channel as the combination of the input heart signals provided by the other input channels. For example, for three input heart signals $A_{raw}$, $B_{raw}$, and $C_{raw}$, using the electrode arrangement as shown in Figure 2, the spatially derived estimates $A_{spatial}$, $B_{spatial}$, and $C_{spatial}$ may be calculated according to the following equations:

$$A_{spatial} = B_{raw} + C_{raw}$$

$$B_{spatial} = A_{raw} - C_{raw}$$

$$C_{spatial} = A_{raw} - B_{raw}.$$

Expressed in other words, the calculation of the spatially derived estimate $A_{spatial}$, $B_{spatial}$, and $C_{spatial}$ is possible because the input channels or sensing vectors use common electrodes. Spatially derived estimates reduce the influence of local electrode noise by creating an alternate representation of the cardiac vector. Each spatially derived estimate $A_{spatial}$, $B_{spatial}$, and $C_{spatial}$ uses two of the raw input channels to estimate using vector mathematics. The spatially derived estimate always operates on the n-1 sample to maintain timing with the temporally derived estimate. The result predicts the value of, for example, the third input channel $C_C$ based on what is sensed at the first and second input channels $C_A$ and $C_B$.

In further embodiments the temporal and spatial estimate calculations may be combined. For example, a spatial estimate may be derived from the temporal estimates. Likewise, a temporal estimate may be derived from spatial estimates.

The estimation unit 10 also calculates from the spatially derived estimate, the temporally derived estimate, and the raw input heart signal of an input channel, respectively, a composite estimate signal $A_{est}$, $B_{est}$, and $C_{est}$, respectively. Expressed in other words, a new filtered estimate of each input channel $C_A$, $C_B$ and $C_C$ is composed of the respective (raw) input heart signals $A_{raw}$, $B_{raw}$ and $C_{raw}$, the temporally derived estimates $A_{temporal}$, $B_{temporal}$, and $C_{temporal}$, and the spatially derived estimates $A_{spatial}$, $B_{spatial}$, and $C_{spatial}$. The following equation shows how the estimates and the raw data might be combined into the composite estimate signal $A_{est}$, $B_{est}$ and $C_{est}$ for each input channel $C_A$, $C_B$, and $C_C$, respectively:

$$A_{est} = A_{raw} + A_{temporal} + A_{spatial}$$

$$B_{est} = B_{raw} + B_{temporal} + B_{spatial}$$

$$C_{est} = C_{raw} + C_{temporal} + C_{spatial}.$$

Combining the three (raw) input heart signals with their estimates helps to reduce random noise and to increase the signal-to-noise ratio.

The implantable medical device 100 further comprises a combination unit 30 that combines the three composite estimates to form a combined SECG output. To reduce the probability that the combining will produce a null result, one estimate channel may be augmented before the final combination. In a further embodiment, a prospect $P_A$, $P_B$, and $P_C$ is calculated for each of the input channels $C_A$, $C_B$, and $C_C$ respectively, using the composite estimate signals $A_{est}$, $B_{est}$, and $C_{est}$, wherein within the calculation of a certain prospect $P_A$, $P_B$ and $P_C$ for an input channel the respective composite estimate signal is augmented by doubling. For example, the calculation of the prospects $P_A$, $P_B$ and $P_C$ can be performed according to the following equations:

$$P_A = 2\,A_{est} + B_{est} + C_{est}$$

$$P_B = A_{est} + 2\,B_{est} + C_{est}$$

$$P_C = A_{est} + B_{est} + 2\,C_{est}$$

$P_A$, $P_B$, and $P_C$ are the prospects and $A_{est}$, $B_{est}$, $C_{est}$ the composite estimate signals of the input channels $C_A$, $C_B$, and $C_C$, respectively. In the preferred embodiment all three prospects are produced simultaneously.

The concept of prospect allows for the selection of at least one prospect as the best suited combined input stream SECG according to selectable criteria such as peak or maximum signal amplitude or signal-to-noise ratio.

In a preferred embodiment, the selection criterion is the maximum signal amplitude of the prospect. In such an embodiment, local and system inherent noise is reduced significantly.

In a further embodiment, a hysteresis is implemented within combination unit 30 in order to reduce the frequency of changing the doubled composite estimate signal, which may distort signal morphology. For example, if after the prospect with $A_{est}$ being doubled is selected as the combined input stream, the prospect with $B_{est}$ doubled meets the prospect selection criteria, the prospect with $B_{est}$ doubled is not immediately used. Rather, prospect with $A_{est}$ being doubled continues to be used as the combined input stream unless the next QRS detection determines the prospect with $B_{est}$ doubled also meets the prospect selection criteria. At that point the prospect with $B_{est}$ doubled would be used as the combined SECG input stream. Preferably the chosen prospect changes only if two consecutive QRS-peak amplitudes lead to the same combined input stream choice.

The combination, in this case, may be expressed using the following equation:

$$Combo = \max(prospect_A, prospect_B, prospect_C).$$

Figure 2A and Figure 2B show representations of electrode placements in embodiments of a cardiac device 100. Electrical signals originating from cardiac activity are sensed by the electrodes 1, 2 and 3 symbolized by the black circles in Figure 2A and Figure 2B. It is understood that the processing of the electrical signals sensed between two electrodes is performed in a known fashion, e.g. for pacemakers for signals sensed by bipolar electrodes implanted in the heart. In particular it is understood that signals sensed between the various electrode combinations described hereafter are processed substantially simultaneously. The three electrodes allow the recording of three input channels, each channel having an electrode in common with each of the other two channels. Referring to Figure 2A and Figure 2B, signals sensed between electrodes 3 and 1 are recorded as input channel $C_A$; signals sensed between electrodes 3 and 2 are recorded as input channel $C_B$ and signals sensed between electrodes 2 and 1 are recorded as input channel $C_C$. In these embodiments, each of the electrodes is shared by two input channels. Expressed in other words, the cardiac device 100 has three input channels $C_A$, $C_B$ and $C_C$ that receive different projections of the heart signal as input heart signals $A_{raw}$, $B_{raw}$ and $C_{raw}$. Each input channel $C_A$, $C_B$ and $C_C$ uses a single pair of the three sensing electrodes and each input channel $C_A$, $C_B$ and $C_C$ senses a different planar projection of the heart signal, wherein the projections are coincident in time. Noise on each input channel $C_A$, $C_B$ and $C_C$ is determined, in part, by the local activity around the sensing electrodes in that particular pair. Combining the three input channels $C_A$, $C_B$ and $C_C$ into a single input channel increases the signal to noise ratio by smoothing some of the random noise associated with each input channel $C_A$, $C_B$ and $C_C$, while emphasizing the QRS-signals, so the QRS complexes.

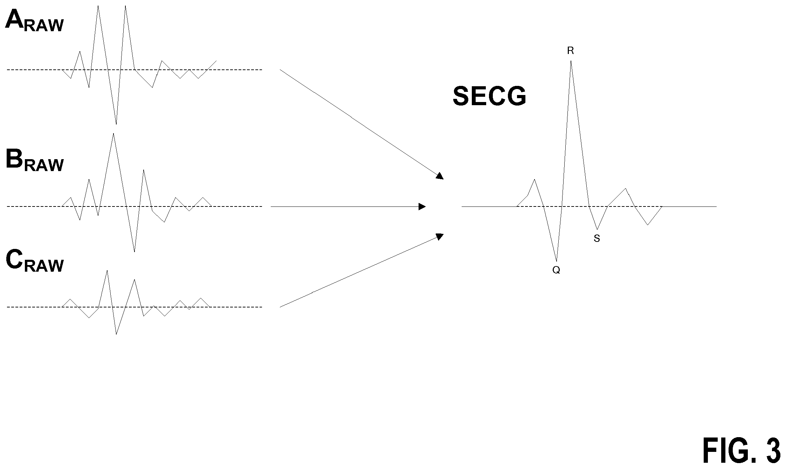

Figure 3 shows a schematic illustration of the combination of three input heart signals $A_{raw}$, $B_{raw}$, and $C_{raw}$ to the eventual combined input stream SECG. As already described, via three different input channels $C_A$, $C_B$ and $C_C$, three input heart signals $A_{raw}$, $B_{raw}$, and $C_{raw}$ are provided which in Figure 3 show three different measurements (planar projections) of the same QRS signal measured from the heart of a living being. These three different input heart signals $A_{raw}$, $B_{raw}$, and $C_{raw}$ are combined to a single data stream, the combined input stream SECG via the calculation of spatial and temporal estimates and the prospects, as described above.

Referring back to Figure 1, the combined input stream may be filtered in an optional filter unit 40 with a high-pass, low-pass, or a pass-through filter, or any combination thereof, before the step of comparing is performed. In one embodiment, the combined input stream SECG is exemplarily passed through two second order Butterworth filters before it is sent to either an automatic sense control unit (ASC) 50 for the detection or to a memory (not shown) for internal storage. Further filtering of the combined input stream SECG removes noise not mitigated by the combination operation performed by the combination unit 30 as already mentioned. Filters can each be configured as high-pass, low-pass or pass-through (delay) filters. As shown, the filter parameters are set by a system adjustment unit 60. They are also stored in a re-programmable area of the cardiac device 100 memory (not shown).

In the preferred embodiment, the filters are exemplarily used as a second order low-pass filter and as a second order high-pass filter each of which introduce a delay of 2 samples. It is also possible to carry out cardiac devices 100, in which the filters are programmed as pass-through filters, with possible delays of 0, 1 or 2 samples. The maximum delay of 2 samples maintains time synchronization between the detection and snapshot versions of the output waveform. The filters exemplarily are arranged in series using a filter loop structure. By arranging the filter as a loop the same hardware may be used to implement each filter, where only the filter coefficients need be changed to enable the type of filter desired. The output of the combination unit 30 passes through each of the filters. The output of the filter loop is sent to either the automatic sense control unit 50 for detection or to a memory (not shown) for internal storage.

In a further embodiment the filter may be configured to output differently filtered signals with one filter parameter set for QRS detection and another filter parameter set for saving and later review. In this way a more faithful reproduction may be viewed by the physician, while a version with very little noise may be used for QRS detection.

The combined signal is input to an automatic sense control unit 50 that is configured to compare the combined input stream (SECG) to an adaptive detection threshold (ATHR), which adapts throughout time. If the combined input stream (SECG) exceeds the adaptive detection threshold (ATHR) a QRS detection is indicated and a corresponding marker signal is generated.

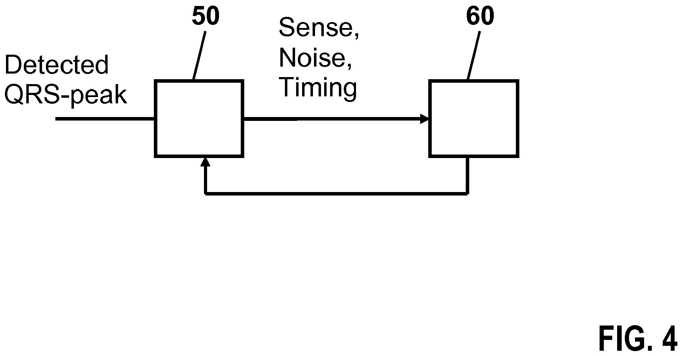

The automatic sense control unit 50 of Figure 1, shown in more detail in Figure 4, outputs specific information about a detection and noise status, as well as about timing that is used by the cardiac device 100. In the preferred embodiment, the cardiac device 100 comprises a system adjustment unit 60 which adjusts the parameters of the automatic sense control unit 50 based on a previous detection, especially based on the detection of a previous QRS-peak, noise condition, and/or timing. In this embodiment, the outputs from the automatic sense control unit 50 are returned to the system adjustment unit 60 to control the combination calculations, for example the determination of which prospect to use.

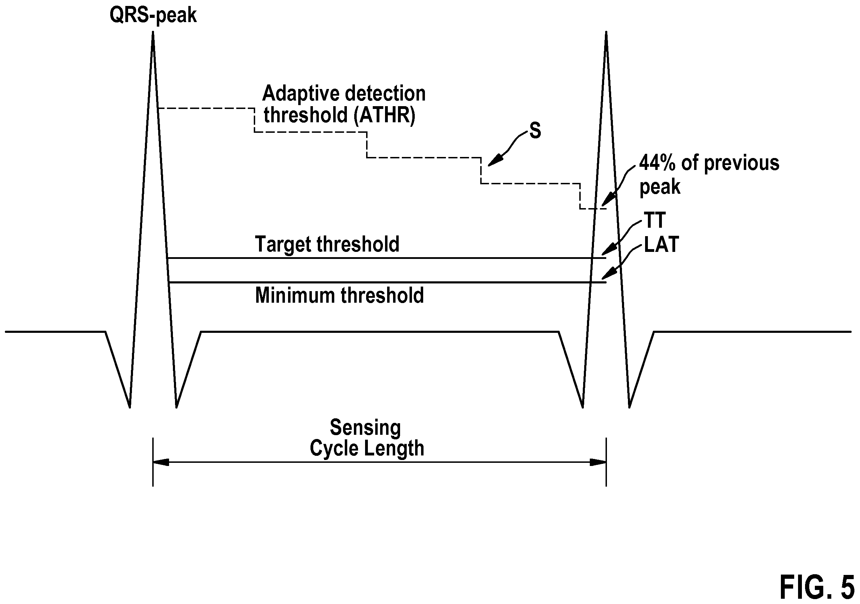

Figure 5 shows a schematic illustration of a threshold adjustment for large amplitude QRS-peaks according to the preferred embodiment. The adaptive detection threshold ATHR is set after each sensing cycle which starts with the detection of a QRS-complex, wherein the value of the adaptive detection threshold ATHR is initially set as a percentage of a QRS-peak amplitude of the combined input stream SECG measured during a sensing cycle. In this embodiment, the adaptive detection threshold ATHR may decrease in steps S during a sensing cycle, until it reaches a target threshold TT, wherein the value of the target threshold TT is also initially set as a percentage of a QRS-peak amplitude of the combined input stream SECG measured during a sensing cycle. The adaptive detection threshold ATHR and the target threshold TT are both greater than or equal to a minimum threshold LAT, and may be set to different percentage values of the QRS-peak amplitude. Expressed in other words, after each detection, the adaptive detection threshold ATHR is set based on the amplitude of the QRS-peak detected within a combined input stream SECG. The adaptive detection threshold ATHR decays in steps S, which are determined by a step duration and a step size, until it reaches the target threshold TT, which is also based on the QRS-peak amplitude. Neither the adaptive detection threshold ATHR nor the target threshold TT is lower than the minimum threshold LAT. The adaptive detection threshold ATHR normally does not decrease beyond the target threshold TT. Furthermore, in the preferred embodiment, the duration of a step S is adjusted based on a moving average of the ventricular cycle length measured from the composite heart signal SECG of a patient. The adaptive detection threshold ATHR steps S may be set such that the ATHR reaches a set amplitude at a point in time when the next QRS peak is expected. In this way, the threshold is maintained above the noise floor as long as possible such that only QRS complexes are detected. In Figure 5, the adaptive detection threshold ATHR exemplarily is initially set equal to an amount of 75% of the QRS-peak amplitude shown on the left side of Figure 5. In this example, after the time of a sensing cycle length has passed the amount of the adaptive detection threshold ATHR has decreased to a value that is equal to 44% of the amount of the previous QRS-peak amplitude.

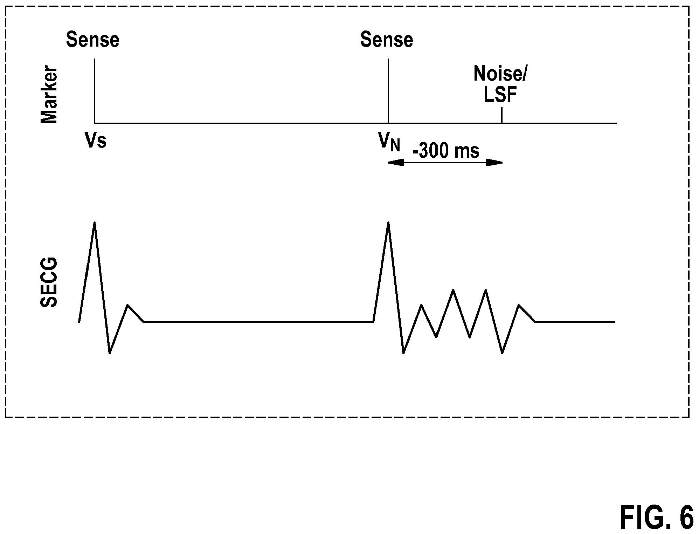

Figure 6 shows a timing diagram for sense and noise markers settable by the cardiac device 100 according to one exemplary embodiment and an exemplary combined input stream SECG showing a QRS complex without noise followed by a QRS complex with noise. Noise conditions can occur only after a sensed event. While the timing diagram is shown in the upper area of Figure 6, the exemplary combined input stream SECG is shown in the lower area of Figure 6. A sense marker is generated when the adaptive detection threshold ATHR is crossed by the combined input stream SECG, which is detected as a valid sensed event Vs and which can be seen on the left side of the upper diagram of Figure 6. On the left side in the lower diagram of Figure 6, the corresponding QRS-peak within the combined input stream SECG is shown, which led to the detection of the valid sensed event Vs. On the right side of the upper diagram of Figure 6, another event is sensed which is identified as an invalid sensed event $V_N$ due to noise following a detected QRS-peak. Within a delay of about 300 ms, if a low signal flag LSF or noise flag is set, which will be described further below, the preceding sensed QRS is determined to be invalid. On the right side of the lower diagram of Figure 6, a QRS-peak followed by noise can be seen within the combined input stream SECG, which led to the setting of the noise marker and the low signal flag LSF. In the preferred embodiment, the low signal flag LSF is available immediately following a detection hold-off period DHP of approximately 300 ms, while the noise flag may be generated within the DHP. If either of the noise markers (Noise or low signal flag LSF) is generated, the preceding sense marker is invalid. A valid sensed event is identified by Vs and an invalid sensed event is identified by $V_N$. Likewise, a valid sensed interval does not include a $V_N$ event.

In the preferred embodiment, a sense detection occurs the first time the combined input stream SECG crosses the adaptive detection threshold ATHR., Three timers are then activated: a peak detection window PDW, a detection hold-off period DHP and a noise sense window NSW. A QRS-peak within the combined input stream SECG is detected during the peak detection window PDW. The nominal settings for this algorithm set the peak detection window PDW and the detection hold-off period DHP to the same nominal length. During the PDW the combined input stream is sampled and the peak value is detected. During the DHP no new events may be sensed and labeled a $V_s$. The NSW is a retriggerable timer that is retriggered whenever a threshold crossing occurs after detection of a new QRS event. If a threshold crossing occurs after DHP and while NSW is active then a noise flag is triggered and the timer resets. No new QRS detection may occur as long athe NSW is active.

Referring back to Figure 5, the adaptive detection threshold ATHR decays over time until it reaches the target threshold TT. If no new sense detection occurs within a specific time the adaptive detection threshold ATHR is set to the current minimum threshold LAT. Expressed differently, the adaptive detection threshold ATHR is set to the minimum threshold LAT of a current sensing cycle, if no new sensing cycle has begun within a specified time.

As already mentioned, the adaptive detection threshold ATHR and the target threshold TT are initially set as a percentage of the previous QRS-peak detected at the end of the peak detection window. The minimum threshold LAT is calculated and set after every QRS-peak detection or after each sense time-out STO. If the minimum threshold LAT is calculated and set after the sense time-out STO then it is not changed after the QRS-peak detection immediately following sense time-out STO. The minimum threshold LAT is calculated and set on subsequent QRS-peak detections and sense time-outs STO.

In the preferred embodiment the minimum threshold LAT is determined by a vector residual of the composite estimate signals $A_{est}$, $B_{est}$, and $C_{est}$. An error $\varepsilon$, which includes different noise components, is calculated, filtered and used for the determination of the minimum threshold LAT, wherein $\varepsilon$ is equal to $\varepsilon = A_{est} - B_{est} - C_{est}$. Moreover, the error $\varepsilon$ is further smoothed, following the equation $\varepsilon_n = \frac{1}{4} |\varepsilon_{HP}| + \frac{3}{4} \varepsilon_{n-1}$, wherein $\varepsilon_{HP}$ is the high-pass filtered error and $\varepsilon_{n-1}$ is the error of the previous sample filtered with a moving average filter, and wherein $\varepsilon_n$ is used for the determination of the minimum threshold LAT. The error signal $\varepsilon$ is expected to be zero when no noise is present. The specific equation to determine $\varepsilon$ depends on the arrangement of the three electrodes and which electrode is the reference for each channel. The determination of the error follows vector math where the sum of the vectors is zero. When noise if present the channels are not as closely related to each other and so the sum of the vectors is determined as the error.

In the preferred embodiment, a low signal flag LSF is set if a detected QRS-peak amplitude of the combined input stream SECG, which is measured during PDW, is lower than a multiple of the amplitude of the minimum threshold LAT. For example a low signal flag may be set if the amplitude of the detected QRS-peak is less than two times LAT. Expressed in other word, the minimum threshold LAT is determined by the composite estimate signals $A_{est}$, $B_{est}$ and $C_{est}$ of the input channels $C_A$, $C_B$ and $C_C$. Using vector mathematics in order to relate the input channels $C_A$, $C_B$ and $C_C$ to each other, the subtraction of the composite estimate signals from each other termed the residual value, so $A_{est} - B_{est} - C_{est}$ theoretically results in a value which is equal to 0. In reality, the subtraction of the sensed signals yields an error that includes both inherent system noise and external uncorrelated noise present on the sensing electrodes. The amplitude of inherent system noise generally does not change. Uncorrelated noise originates locally, is not present at all the sensing electrodes and can be associated with muscle potentials or sensing electrode rubbing. The error is used to dynamically determine the minimum threshold LAT and is derived from the three composite estimate signals $A_{est}$, $B_{est}$ and $C_{est}$ as described above, wherein $\varepsilon$ is equal to an error signal resulting from the subtraction of the three composite estimate signals $A_{est}$, $B_{est}$ and $C_{est}$. The error signal $\varepsilon$ is then high-pass filtered with the same filter settings as the combined input stream SECG used for the QRS-peak detection resulting in a high-pass filtered error signal $\varepsilon_{HP}$. The error signal is further filtered with a moving average filter according to the following equation:

$$\varepsilon_n = \frac{1}{4}\left|\varepsilon_{HP}\right| + \frac{3}{4}\varepsilon_{n-1},$$

wherein $\varepsilon_n$ is equal to the error signal of a current sample filtered with a moving average filter. Over a predetermined amount of time, the maximum of the averaged absolute value of the error signal of a current sample filtered with a moving average filter $\varepsilon_n$ is calculated according to the following equation:

$$\varepsilon_{max} = \max(\varepsilon_n)\big|_1^m,$$

wherein m is the number of samples that occur between subsequent QRS-peak detections or sense time-outs STO.

$\varepsilon_{max}$ is acquired over a deterministic time period, especially between sensed QRS-peaks or sense time-outs STO, but it may also be acquired over set time periods not related to QRS detections. In the preferred embodiment, a constant multiplier k is used to adjust the amplitude of the minimum threshold LAT, which may be necessary due to preconditioning of the combined input stream SECG. The nominal value for k may be set to maximize the number of Vs events while minimizing the number of low signal flags, or k may be set to a value that compensates for the decrease in amplitude due to filtering the error signal. For example, k may be in the range of 1 to 4. The constant k is further limited, such that, the scaled value of $\varepsilon_{max}$ varies between a range of values appropriate for the system. An example of which is shown in the following equation in order to avoid the minimum threshold LAT from becoming too sensitive or too insensitive.

$$LAT = 14 \leq k \cdot \varepsilon_{\max} \leq 21.$$

Because the minimum threshold LAT is derived from the three composite estimate signals $A_{est}$, $B_{est}$, and $C_{est}$ and because it is constantly changing, it provides a more accurate reference to reliably classify low signals. If the target threshold TT is less than the minimum threshold LAT, it is set to the minimum threshold LAT. In this way the adaptive threshold ATHR is maintained above the random sensed noise. The above calculations occur for every sample n of the combined input stream SECG received. The maximum of the averaged absolute value of the error signal of a current sample filtered with a moving average filter $\varepsilon_{\max}$ may be reset after each sense detection, QRS-peak detection, or sense time-out STO such that a new $\varepsilon_{\max}$ can be calculated.

In the preferred embodiment, noise in the combined input stream SECG is detected in two ways: noise condition NCND from the automatic sense control unit 50 and low signal flag LSF. The detection of a QRS-peak with very small amplitude, indicated by an amplitude less than a multiple of LAT, is treated as noise because it is deemed to be unreliable (see Figure 6). The noise condition NCND uses, for example, the target threshold TT to determine a threshold crossing while the low signal flag LSF uses the minimum threshold LAT to determine a difference in the amplitude of the combined input signal SECG. NCND may use other threshold values such as ATHR or an independent threshold derived from a feature in the SECG stream. In the preferred embodiment, the noise detection uses a noise condition NCND interrupt from the automatic sense control unit 50. The noise condition NCND is set if the combined input signal crosses the target threshold TT while the noise sense window NSW is still active after the detection hold-off period DHP. No new sensed event can occur until the noise sense window NSW times out.

The low signal flag LSF is set when the QRS-peak of a detected QRS complex is too low to be considered reliable, which could happen if a QRS complex has a low amplitude or if noise caused the detection. The low signal flag LSF is set or cleared upon comparing the QRS-peak amplitude to the minimum threshold LAT following the peak detection window PDW.

If the QRS-peak of the detected signal is less than a low signal threshold, which in this embodiment is equal to or less than two times the amount of the minimum threshold LAT, then the detected QRS-peak is declared a low signal. In this way, if noise decreases, thereby lowering the minimum threshold LAT, the QRS-peak amplitude of a valid sensed event Vs is also allowed to decrease.

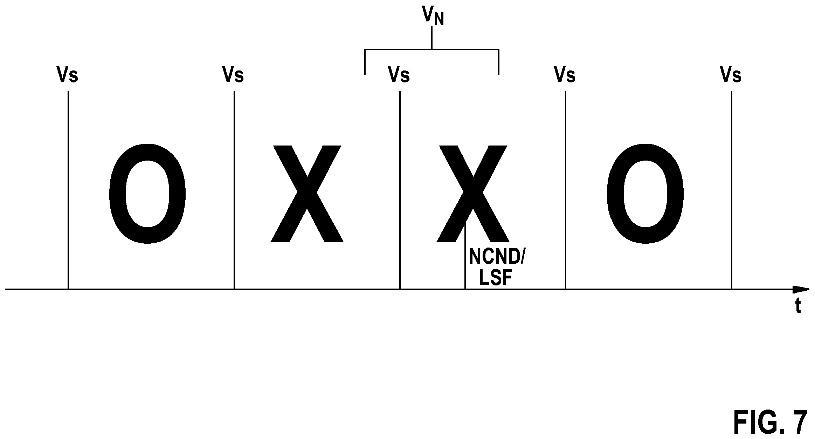

As already described, the decay time of the adaptive detection threshold ATHR is adjusted after every sensed event based on a moving average of the ventricular cycle length measured from the heart of a living being. The average cycle length ACL is updated only after valid sensed events Vs are detected. Figure 7 shows intervals used for the calculation of average cycle length ACL according to one exemplary embodiment of the present invention. It shows a progression of sensed events, both valid and invalid (Vs and $V_N$), and it further shows which intervals are used to calculate the average cycle length ACL. The intervals with an "O" are used and are bracketed by valid sensed events Vs events, while intervals including an invalid sensed event $V_N$ are not included, which is indicated by an "X" in Figure 7. Moreover, a noise marker is shown in Figure 7, indicating an invalid detection, causing that the respective intervals are not used for the calculation of the average cycle length ACL.

As already described for Figure 5, the adaptive detection threshold ATHR starts at its maximum with a value that is equal to a fraction of a detected QRS-peak. The adaptive detection threshold ATHR decays in steps until it reaches the target threshold TT. In one embodiment, each step reduces the adaptive detection threshold ATHR by 12.5%. The target threshold TT is equal to a fraction of the same QRS-peak that was used for the determination of the initial value of the adaptive detection threshold ATHR. An exemplary adaptive detection threshold ATHR can not fall below the minimum threshold LAT. Furthermore, the maximum, initial value of the adaptive detection threshold ATHR is equal to 87.5% of the QRS-peak detected while the value of the target threshold TT is equal to 25% of the QRS-peak. In this embodiment of the method, the step duration of the decay of the adaptive detection threshold ATHR is adjusted such that the adaptive detection threshold ATHR reaches 44% of the detected QRS-peak amplitude at the average cycle length ACL in time. Therefore, as the heart rate of the heart of the living being the measurements are performed on increases or decreases, the adaptive detection threshold ATHR always decays to 44% when the next QRS complex is expected. This maintains the adaptive detection threshold ATHR above possible noise that would be present between QRS complexes, for example T-waves, muscle noise, and the like.

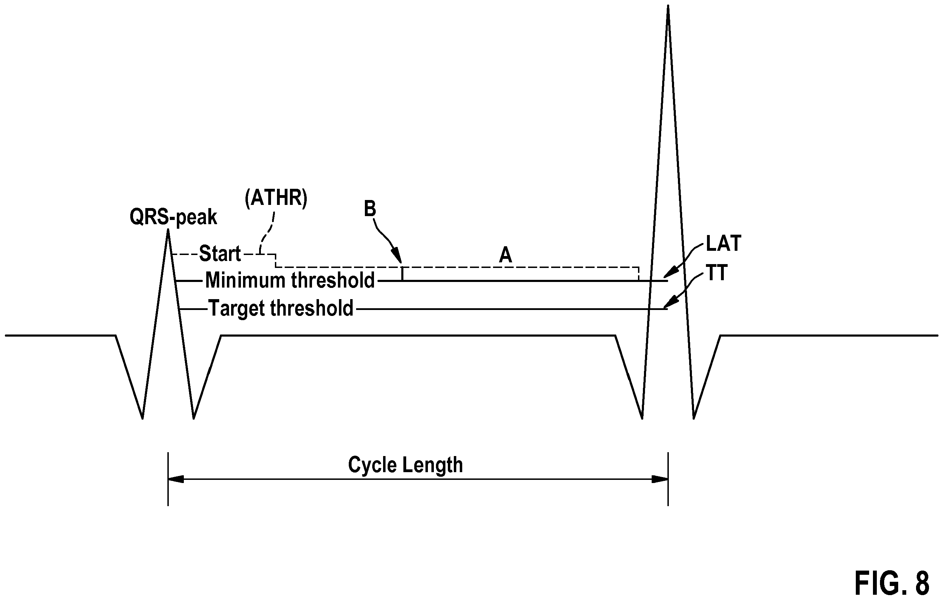

Figure 8 shows a schematic illustration of a threshold adjustment of the adaptive detection threshold ATHR for small amplitude QRS-peaks according to one exemplary embodiment. Therefore Figure 8 shows a special situation when the amplitude of a QRS-peak is small but still valid because it is not classified as a low signal. In this case, the estimated target threshold TT can be lower than the minimum threshold LAT if the amplitude of a detected QRS-peak is less than four times the amount of the minimum threshold LAT, when TT is set to 25% of the detected QRS-peak. The adaptive detection threshold ATHR would then only decrease until it reached the minimum threshold LAT, which is greater than the predicted target threshold TT, and would cause the adaptive detection threshold ATHR to decay too rapidly to the minimum threshold LAT before the average cycle length ACL, which is shown by line B. Therefore, in this case, the time per decay step is calculated based on the difference between the initial value of the adaptive detection threshold ATHR and the value of the minimum threshold LAT, such that the adaptive detection threshold ATHR decays to the minimum threshold LAT at an average cycle length ACL in time, which is shown by line A. Therefore, the method maintains the adaptive detection threshold ATHR above possible noise while decaying fast enough to detect the next expected heart beat. The decay of the adaptive detection threshold ATHR adapts to changes in the heart rate and to changes in the amplitude of the QRS-peak. It should be noted that the average cycle length ACL does not include any intervals that include invalid sense detections, as already mentioned further above.

**Claims**

1. A method for the detection of QRS signals, comprising the steps of:

    - providing at least two input heart signals via at least two separate input channels, wherein each channel is connected to pairs of sensing electrodes (1, 2, 3), wherein the at least two input heart signals are recorded by said pairs of sensing electrodes and provided coincidental in time;
    - generating estimated signals from the at least two separate input heart signals, in such a way that for the combination of input heart signals provided by different input channels, the signal to noise ratio is increased by smoothing some of the random noise associated with each input channel;

    wherein the estimated signals are spatially derived estimates and/or temporally derived estimates, wherein the temporally derived estimate for an input channel is a smoothed value calculated from samples of the input heart signal of an input channel;

    - combining the estimated signals from the at least two separate input heart signals to a combined input stream (SECG);
    - detecting the QRS signal by comparing the combined input stream (SECG) to an adaptive detection threshold (ATHR) which adapts throughout time.

2. The method according to claim 1, wherein providing at least two input heart signals via at least two separate input channels comprises: providing at least three input heart signals via at least three separate input channels; wherein the spatially derived estimate of an input channel is calculated as a combination of the input heart signals provided by the at least two other input channels.

3. The method according to claim 1 and 2, wherein the step of combining the estimated signals to a combined input stream (SECG) further comprises the step of:

    - combining the spatially derived estimate, the temporally derived estimate and the input heart signal of an input channel for each input channel respectively to provide a composite estimate signal for each input channel respectively.

4. The method according to claim 3, wherein the step of combining the estimated signals to a combined input stream (SECG) further comprises the steps of:

    - calculating a prospect P for each input channel, wherein P is calculated using the composite estimate signals, wherein within the calculation of a certain P for a channel, the respective composite estimate signal calculated for the respective channel is augmented;
    - choosing at least one P as the combined input stream (SECG).

5. The method according to one of the previous claims, wherein the adaptive detection threshold (ATHR) is set after each detection of a QRS signal and wherein the value of the adaptive detection threshold (ATHR) is initially set as a percentage of a QRS-peak amplitude of the combined input stream (SECG) measured during a detection of a QRS signal and wherein the adaptive detection threshold (ATHR) decreases throughout time.

6. The method according to claim 5, wherein the adaptive detection threshold (ATHR) decreases in steps (S) until it reaches a target threshold (TT), wherein the duration of a step (S) is adjusted based on a moving average of the ventricular cycle length measured from the composed heart signal of the body from which the combined input stream (SECG) is measured.

7. The method according to claim 6, wherein the value of the target threshold (TT) is initially set as a percentage of a QRS-peak amplitude of the combined input stream (SECG) measured during a detection of a QRS signal and wherein the adaptive detection threshold (ATHR) and the target threshold (TT) are always greater than or equal to a minimum threshold (LAT).

8. The method according to claim 3 and one of the claims 6 to 7, wherein the minimum threshold (LAT) is determined by the composite estimate signals, wherein a vector residual of the composite estimate signals is used for the determination of the minimum threshold (LAT).

9. The method according to one of the claims 6 to 8, wherein a low signal flag (LSF) is set if a detected QRS-peak amplitude of the combined input stream (SECG) is lower than a scaled value of the minimum threshold (LAT).

10. The method according to one of the previous claims, wherein of the pairs of sensing electrodes one electrode is shared by two of the input channels.

11. A cardiac device (100) for use in detecting QRS signals within a composite heart signal of a body, comprising:

- an input unit (10) to which at least three sensing electrodes (1, 2, 3) are attachable to provide at least two input heart signals recorded by pairs of sensing electrodes via at least two separate input channels provided coincidental in time;
- an estimation unit (20), configured to generate estimated signals from the at least two separate input heart signals, in such a way that for the combination of input heart signals provided by different input channels, the signal to noise ratio is increased by smoothing some of the random noise associated with each input channel;

wherein the estimated signals are spatially derived estimates and/or temporally derived estimates, wherein the temporally derived estimate for an input channel is a smoothed value calculated from samples of the input heart signal of an input channel;

- a combination unit (30), configured to combine the estimated signals from the at least two separate input heart signals to a combined input stream (SECG);

- an automatic sense control unit (50), configured to compare the combined input stream (SECG) to an adaptive detection threshold (ATHR) which adapts throughout time;

- a system adjustment unit (60), configured to adjust at least one parameter of the automatic sense control unit (50);

wherein the cardiac device (100) is adapted to execute the method according to at least one of the previous claims.

12. The cardiac device (100) according to claim 11, further comprising:

- at least one filter unit, configured to filter the at least two input heart signals and/or the combined input stream (SECG).

13. The cardiac device according to claim 11, wherein of the pairs of sensing electrodes one electrode is shared by two of the input channels.

**Patentansprüche**

1. Verfahren zum Erfassen von QRS-Signalen, das folgende Schritte umfasst:

- Liefern von mindestens zwei Eingangsherzsignalen über mindestens zwei separate Eingangskanäle, wobei jeder Kanal mit Paaren von Messelektroden (1, 2, 3) verbunden ist, wobei die mindestens zwei Eingangsherzsignale von den Paaren von Messelektroden aufgezeichnet werden und zeitlich übereinstimmend geliefert werden;

- Erzeugen geschätzter Signale aus den mindestens zwei separaten Eingangsherzsignalen derart, dass für die Kombination aus Eingangsherzsignalen, die von unterschiedlichen Eingangskanälen geliefert werden, das Signal-Rausch-Verhältnis durch Glätten eines Teils des weißen Rauschens erhöht wird, das jedem Eingangskanal zugehörig ist;

wobei die geschätzten Signale räumlich abgeleitete Schätzungen und/oder zeitlich abgeleitete Schätzungen sind, wobei die zeitlich abgeleitete Schätzung für einen Eingangskanal ein geglätteter Wert ist, der aus Abtastwerten des Eingangsherzsignals eines Eingangskanals berechnet wird;

- Kombinieren der geschätzten Signale aus den mindestens zwei separaten Eingangsherzsignalen zu einem kombinierten Eingangsstrom (SECG);

- Erfassen des QRS-Signals durch Vergleichen des kombinierten Eingangsstroms (SECG) mit einer adaptiven Erfassungsschwelle (ATHR), die sich über die gesamte Zeit anpasst.

2. Verfahren nach Anspruch 1, wobei das Liefern von mindestens zwei Eingangsherzsignalen über mindestens zwei separate Eingangskanäle Folgendes umfasst:

Liefern von mindestens drei Eingangsherzsignalen über mindestens drei separate Eingangskanäle;
wobei die räumlich abgeleitete Schätzung eines Eingangskanals als Kombination aus den Eingangsherzsignalen berechnet wird, die von den mindestens zwei anderen Eingangskanälen geliefert werden.

3. Verfahren nach Anspruch 1 und 2, wobei der Schritt des Kombinierens der geschätzten Signale zu einem kombinierten Eingangsstrom (SECG) ferner folgenden Schritt umfasst:

- Kombinieren der räumlich abgeleiteten Schätzung, der zeitlich abgeleiteten Schätzung und des Eingangsherzsignals eines Eingangskanals jeweils für jeden Eingangskanal zum Liefern eines zusammengesetzten Schätzungssignals jeweils für jeden Eingangskanal.

4. Verfahren nach Anspruch 3, wobei der Schritt des Kombinierens der geschätzten Signale zu einem kombinierten Eingangsstrom (SECG) ferner folgende Schritte umfasst:

- Berechnen einer Variablen P für jeden Eingangskanal, wobei P unter Verwendung der zusammengesetzten Schätzungssignale berechnet wird, wobei innerhalb der Berechnung eines bestimmten P für einen Kanal das jeweilige zusammengesetzte Schätzungssignal, das für den jeweiligen Kanal berechnet wird, verstärkt wird;
- Auswählen von mindestens einem P als kombiniertem Eingangsstrom (SECG).

5. Verfahren nach einem der vorhergehenden Ansprüche, wobei die adaptive Erfassungsschwelle (ATHR) nach jedem Erfassen eines QRS-Signals festgelegt wird und wobei der Wert der adaptiven Erfassungsschwelle (ATHR) anfangs als Prozentsatz einer QRS-Peakamplitude des kombinierten Eingangsstroms (SECG) festgelegt wird, die während einer Erfassung eines QRS-Signals gemessen wird, und wobei die adaptive Erfassungsschwelle (ATHR) über die gesamte Zeit sinkt.

6. Verfahren nach Anspruch 5, wobei die adaptive Erfassungsschwelle (ATHR) in Stufen (S) abnimmt, bis sie eine Zielschwelle (TT) erreicht, wobei die Dauer einer Stufe (S) auf der Grundlage eines gleitenden Durchschnitts der ventrikulären Zykluslänge angepasst wird, die aus dem zusammengesetzten Herzsignal des Körpers gemessen wird, aus dem der kombinierte Eingangsstrom (SECG) gemessen wird.

7. Verfahren nach Anspruch 6, wobei der Wert der Zielschwelle (TT) anfangs als Prozentsatz einer QRS-Peakamplitude des kombinierten Eingangsstroms (SECG) festgelegt wird, die während einer Erfassung eines QRS-Signals gemessen wird, und wobei die adaptive Erfassungsschwelle (ATHR) und die Zielschwelle (TT) stets größer gleich einer Mindestschwelle (LAT) sind.

8. Verfahren nach Anspruch 3 und einem der Ansprüche 6 bis 7, wobei die Mindestschwelle (LAT) durch die zusammengesetzten Schätzungssignale bestimmt wird, wobei ein Vektorrest der zusammengesetzten Schätzungssignale zur Bestimmung der Mindestschwelle (LAT) verwendet wird.

9. Verfahren nach einem der Ansprüche 6 bis 8, wobei eine Markierung für einen niedrigen Signalpegel (LSF) gesetzt wird, wenn eine erfasste QRS-Peakamplitude des kombinierten Eingangsstroms (SECG) geringer ist als ein skalierter Wert der Mindestschwelle (LAT).

**10.** Verfahren nach einem der vorhergehenden Ansprüche, wobei von den Paaren von Messelektroden eine Elektrode von zwei der Eingangskanäle gemeinsam genutzt wird.

**11.** Herzvorrichtung (100) zur Verwendung beim Erfassen von QRS-Signalen in einem zusammengesetzten Herzsignal eines Körpers, umfassend:

- eine Eingangseinheit (10), an der mindestens drei Messelektroden (1, 2, 3) anbringbar sind, damit sie mindestens zwei zeitlich übereinstimmend gelieferte Eingangsherzsignale, die von Paaren von Messelektroden aufgezeichnet werden, über mindestens zwei separate Eingangskanäle liefert;
- eine Schätzeinheit (20), die so eingerichtet ist, dass sie geschätzte Signale aus den mindestens zwei separaten Eingangsherzsignalen derart erzeugt, dass für die Kombination aus Eingangsherzsignalen, die von unterschiedlichen Eingangskanälen geliefert werden, das Signal-Rausch-Verhältnis durch Glätten eines Teils des weißen Rauschens erhöht wird, das jedem Eingangskanal zugehörig ist;

wobei die geschätzten Signale räumlich abgeleitete Schätzungen und/oder zeitlich abgeleitete Schätzungen sind, wobei die zeitlich abgeleitete Schätzung für einen Eingangskanal ein geglätteter Wert ist, der aus Abtastwerten des Eingangsherzsignals eines Eingangskanals berechnet wird;

- eine Kombinationseinheit (30), die so eingerichtet ist, dass sie die geschätzten Signale aus den mindestens zwei separaten Eingangsherzsignalen zu einem kombinierten Eingangsstrom (SECG) kombiniert;
- eine automatische Messsteuereinheit (50), die so eingerichtet ist, dass sie den kombinierten Eingangsstrom (SECG) mit einer adaptiven Erfassungsschwelle (ATHR) vergleicht, die sich über die gesamte Zeit anpasst;
- eine Systemeinstelleinheit (60), die so eingerichtet ist, dass sie mindestens einen Parameter der automatischen Messsteuereinheit (50) einstellt;

wobei die Herzvorrichtung (100) so ausgelegt ist, dass sie das Verfahren nach mindestens einem der vorhergehenden Ansprüche ausführt.

**12.** Herzvorrichtung (100) nach Anspruch 11, ferner umfassend:

- mindestens eine Filtereinheit, die so eingerichtet ist, dass sie die mindestens zwei Eingangsherzsignale und/oder den kombinierten Eingangsstrom (SECG) filtert.

**13.** Herzvorrichtung nach Anspruch 11, wobei von den Paaren von Messelektroden eine Elektrode von zwei der Eingangskanäle gemeinsam genutzt wird.

**Revendications**

**1.** Procédé pour la détection de signaux QRS, comprenant les étapes de :

- mise en place d'au moins deux signaux cardiaques d'entrée via au moins deux canaux d'entrée séparés, où chaque canal est relié à des paires d'électrodes de détection (1, 2, 3), où les au moins deux signaux cardiaques d'entrée sont enregistrés par lesdites paires d'électrodes de détection et délivrent une coïncidence dans le temps ;
- génération des signaux estimés à partir des au moins deux signaux cardiaques d'entrée séparés, de façon à ce que, pour la combinaison des signaux cardiaques d'entrée délivrés par les différents canaux d'entrée, le ratio signal sur bruit soit augmenté par un lissage de certains bruits aléatoires associés à chaque canal d'entrée ;

où les signaux estimés sont des estimations dérivées spatialement et/ou des estimations dérivées temporellement, où l'estimation dérivée temporellement pour un canal d'entrée est une valeur lissée calculée à partir d'échantillons du signal cardiaque d'entrée d'un canal d'entrée ;

- la combinaison des signaux estimés à partir des au moins deux signaux cardiaques d'entrée séparés en un flux d'entrée combiné (SECG) ;
- la détection du signal QRS par comparaison du flux d'entrée combiné (SECG) avec un seuil de détection adaptatif (ATHR) qui s'adapte au fil du temps.

**2.** Procédé selon la revendication 1, dans lequel la délivrance d'au moins deux signaux cardiaques d'entrée via au moins deux canaux d'entrée séparés comprend: la délivrance d'au moins trois signaux cardiaques d'entrée via au moins trois canaux d'entrée séparés ;

où l'estimation dérivée spatialement d'un canal d'entrée est calculée en tant que combinaison des signaux cardiaques d'entrée délivrés par les au moins deux autres canaux d'entrée.

**3.** Procédé selon la revendication 1 ou 2, dans lequel l'étape de combinaison des signaux estimés en un flux d'entrée combiné (SECG) comprend en outre l'étape de :

- combinaison de l'estimation dérivée spatialement, de l'estimation dérivée temporellement et du signal cardiaque d'entrée d'un canal d'entrée pour chaque canal d'entrée respectivement pour délivrer un signal estimé composite pour chaque canal d'entrée respectivement.

**4.** Procédé selon la revendication 3, dans lequel l'étape de combinaison des signaux estimés en un flux d'entrée combiné (SECG) comprend en outre les étapes de :

- calcul d'un prospect P pour chaque canal d'entrée, où P est calculé en utilisant les signaux estimés composites, où dans le calcul d'un certain P pour un canal, le signal estimé composite respectif calculé pour le canal respectif est augmenté_;
- choix d'au moins un P en tant que flux d'entrée combiné (SECG).

**5.** Procédé selon l'une des revendications précédentes, dans lequel le seuil de détection adaptatif (ATHR) est établi après chaque détection d'un signal QRS et où la valeur du seuil de détection adaptatif (ATHR) est initialement établie en tant que pourcentage d'une amplitude du pic QRS du flux d'entré combiné (SECG) mesurée au cours de la détection d'un signal QRS et où le seuil de détection adaptatif (ATHR) diminue au fil du temps.

**6.** Procédé selon la revendication 5, dans lequel le seuil de détection adaptatif (ATHR) diminue dans les étapes (S) jusqu'à ce qu'il atteigne un seuil cible (TT), où la durée d'une étape (S) est ajustée sur la base d'une moyenne mobile de la longueur du cycle ventriculaire mesurée à partir du signal cardiaque composé du corps à partir duquel le flux d'entrée combiné (SECG) est mesuré.

**7.** Procédé selon la revendication 6, dans lequel la valeur du seuil cible (TT) est initialement établie en tant que pourcentage d'une amplitude du pic QRS du flux d'entrée combiné (SECG) mesurée au cours d'une détection d'un signal QRS et où le seuil de détection adaptatif (ATHR) et le seuil cible (TT) sont toujours supérieurs ou égaux à un seuil minimum (LAT).

**8.** Procédé selon la revendication 3 et l'une des revendications 6 à 7, dans lequel le seuil minimum (LAT) est déterminé par les signaux estimés composites, où un vecteur résiduel des signaux estimés composites est utilisé pour la détermination du seuil minimum (LAT).

**9.** Procédé selon l'une des revendications 6 à 8, dans lequel un drapeau de signalisation de niveau bas (LSF) est établi si une amplitude du pic QRS du flux d'entrée combiné (SECG) est inférieure à une valeur étalonnée du seuil minimum (LAT).

**10.** Procédé selon l'une des revendications précédentes, dans lequel, parmi les paires d'électrodes de détection, une électrode est partagée par deux des canaux d'entrée.

**11.** Dispositif cardiaque (100) pour une utilisation dans la détection des signaux QRS dans un signal cardiaque composite d'un corps, comprenant :

- une unité d'entrée (100) à laquelle au moins trois électrodes de détection (1, 2, 3) sont attachées pour délivrer au moins deux signaux cardiaques d'entrée enregistrés par des paires d'électrodes de détection via au moins deux canaux d'entrée séparés délivrant une coïncidence dans le temps ;
- une unité d'estimation (20), configurée pour générer des signaux estimés à partir des au moins deux signaux cardiaques d'entrée séparés, de façon à ce que pour la combinaison des signaux cardiaques d'entrée délivrés par les différents canaux d'entrée, le ratio signal sur bruit soit augmenté par le lissage de certains bruits aléatoires associés à chacun des canaux d'entrée ;

où les signaux estimés sont des estimations dérivées spatialement et/ou des estimations dérivées temporellement, où l'estimation dérivée temporellement pour un canal d'entrée est une valeur lissée calculée à partir d'échantillons du signal cardiaque d'entrée d'un canal d'entrée ;

- une unité de combinaison (30), configurée pour combiner les signaux estimés à partir des au moins deux signaux cardiaques d'entrée séparés en un flux d'entrée combiné (SECG) ;
- une unité de contrôle de la détection automatique (50), configurée pour comparer le flux d'entrée combiné (SECG) avec un seuil de détection adaptatif (ATHR) qui s'adapte au fil du temps ;
- une unité d'ajustement du système (60), configurée pour ajuster au moins un paramètre de l'unité de contrôle de la détection automatique (50) ;

où le dispositif cardiaque (100) est adapté pour exécuter le procédé selon au moins l'une des revendications précédentes.

12. Dispositif cardiaque (100) selon la revendication 11, comprenant en outre :

- au moins une unité de filtres, configurée pour filtrer les au moins deux signaux cardiaques d'entrée et/ou le flux d'entrée combiné (SECG).

13. Dispositif cardiaque selon la revendication 11, dans lequel, parmi les paires d'électrodes de détection, une électrode est partagée par deux des canaux d'entrée.

**FIG. 1**

EP 2 767 224 B1

FIG. 2A

FIG. 2B

EP 2 767 224 B1

EP 2 767 224 B1

$A_{RAW}$

$B_{RAW}$

$C_{RAW}$

SECG

R

Q

S

FIG. 3

FIG. 4

FIG. 5

EP 2 767 224 B1

FIG. 6

EP 2 767 224 B1

FIG. 7

EP 2 767 224 B1

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4458692 A

- US 5967994 A